# EUROPEAN PATENT SPECIFICATION

(11) **EP 2 836 176 B1**
(45) Date of publication and mention of the grant of the patent: **23.08.2017**
(21) Application number: 13736874.2
(22) Date of filing: 08.07.2013
(51) Int. Cl.: A61F 9/008

(54) **TECHNIQUE FOR TREATING PRESBYOPIA**
VERFAHREN ZUR BEHANDLUNG VON PRESBYOPIE
TECHNIQUE POUR TRAITER LA PRESBYTIE

(43) Date of publication of application: 18.02.2015
(73) Proprietor: WaveLight GmbH, 91058 Erlangen (DE)
(72) Inventor: SEILER, Theo, 8002 Zuerich (CH); DONITZKY, Christof, 90542 Eckental (DE)
(74) Representative: Frenkel, Matthias Alexander
(86) International application number: PCT/EP2013/064399
(87) International publication number: WO 2015/003739

(56) References cited:
- WO-A1-2009/058755
- WO-A2-2009/123700
- US-A1- 2008 281 304
- US-A1- 2012 033 182

## Description

The present disclosure is concerned with techniques for reshaping a human cornea to treat presbyopia.

A frequently employed technique for eliminating visual defects of the human eye (such as e.g., myopia or hyperopia or astigmatism) is referred to as LASIK (Laser in-situ Keratomileusis). LASIK is a technique in which a small cover disc in the cornea is cut free, which cover disc remains connected to surrounding corneal tissue through a hinge, so that the cover disc can be folded aside to expose the underlying tissue regions of the cornea and can be folded back following an ablation process performed on the exposed tissue regions using UV laser radiation. The cover disc is conventionally referred to as a flap in the related art. On account of the removal of stromal material by the ablation process, a changed shape of the anterior surface of the cornea results after the flap has been folded back. The changed shape of the anterior corneal surface results in a different refractive behavior of the cornea and consequently of the overall imaging system of the eye. Suitable definition of the ablation pattern ensures that the initial visual defect is at least distinctly attenuated and, at best, is almost completely eliminated.

For purpose of this disclosure it is assumed hereinafter that the skilled reader is well-familiar with the LASIK technique.

Presbyopia is a well-known phenomenon due to aging. Presbyopia is caused by a loss of elasticity and/or by alterations of the core of the human lens such that the accommodation amplitude decreases with increasing age of the patient. This results in a loss of sharp vision in the near range.

According to the conventional art, presbyopia can be treated with the use of glasses having multifocal lenses (so-called multifocals). It is also known in the art to subject a presbyopic eye to a surgical operation to implant an intraocular lens (IOL) with multifocal characteristics.

Techniques related to finding a customized ablation target using both wavefront eye refractor and corneal topography information, wherein the treated eye may be left with a residual error and wherein ray tracing may be used to account for oblique surface effects and transverse wavefront propagation effects are described in the US 2008/0281304 A1.

In the field of ophthalmic refractive surgery, ray tracing is a technique that has attained increased attention in recent times. Ray tracing can be used to determine an ablation pattern defining an amount of corneal tissue to be removed in the course of a laser-surgical operation in order to reshape the anterior surface of the cornea for the purpose of reducing aberrations of the eye (visual defects). Ray tracing is usually based on an eye model (e.g., Gullstrand, Navarro, Liou and Brennan, Emsley et al.) in conjunction with measured data of the specific eye to be treated, wherein the measured data may relate to the anterior and/or posterior surface of the cornea, the depth of the anterior chamber of the eye, the dimension of the lens, the anterior and/or posterior surface of the lens, the length of the eye, data regarding the retina, and the measured wavefront of the entire eye.

An ablation pattern obtained through ray tracing is individual for the patient's eye and can be used to control a system for reshaping the cornea of the patient's eye wherein the system is controlled by a computer programmed accordingly.

Embodiments of the present disclosure are aimed at providing a technique for reshaping a cornea of a human patient's eye with the aim of treating presbyopia. In this context, treating presbyopia means that the treatment achieves an improvement for at least of one near and distance vision of the patient.

In one aspect, the present disclosure provides a method of determining a corneal ablation pattern for treating presbyopia of a human eye, comprising: defining a non-zero amount of induced aberration for a presbyopic human eye; and applying a ray tracing process to determine a corneal ablation pattern defining an amount of corneal tissue that, when removed from the eye, leaves the eye with the defined amount of induced aberration.

In another aspect, the present disclosure provides a method of treating presbyopia of a human eye, comprising: defining a non-zero amount of induced aberration for the eye; applying a ray tracing process to determine a corneal ablation pattern defining an amount of corneal tissue that, when removed from the eye, leaves the eye with the defined amount of induced aberration; and moving a focal point of laser radiation over an exposed surface of corneal tissue of the eye to remove corneal tissue in accordance with the determined ablation pattern.

In yet another aspect, the present disclosure provides a method of generating a control program for an ophthalmic laser apparatus, the control program containing instructions that, when executed by a control computer of the apparatus, control an optical system of the apparatus to move focused laser radiation over a target, the method comprising steps of: defining a non-zero amount of induced aberration for a presbyopic human eye; applying a ray tracing process to determine a corneal ablation pattern defining an amount of corneal tissue that, when removed from the eye, leaves the eye with the defined amount of induced aberration; and generating instructions for the computer program in accordance with the determined ablation pattern.
In still another aspect, the present disclosure provides a computer program product containing instructions that, when executed by a computer, perform a ray tracing process to determine a corneal ablation pattern defining an amount of corneal tissue that, when removed from a presbyopic human eye, leaves the eye with a pre-defined non-zero amount of induced aberration; and control a laser device to perform ablation on the eye in accordance with the determined ablation pattern.
In certain embodiments, the induced aberration includes at least one of sphere, spherical aberration, coma, and astigmatism. In certain embodiments, the induced aberration includes even higher-order aberration.
In certain embodiments, the ray tracing process is performed using measurement data for one or more of the following: pre-operative refraction of the eye, pre-operative aberration of the eye, corneal topography of the eye, one or more opto-geometrical parameters of the eye, one or more pupil-related parameters.
In embodiments of the present disclosure, the induced aberration is effective to increase a depth of field of the eye by about 1 to 3 diopters. According to a certain example the induced aberration is effective to increase a depth of field of the eye by about 2 diopters.
The present disclosure also encompasses a system for reshaping a cornea of a patient's eye to treat presbyopia, the system comprising a source for emitting laser radiation having radiation properties suitable for ablating corneal tissue from the eye; optical components for guiding and focussing the emitted laser radiation onto the cornea; and a computer for controlling the optical components in accordance with an ablation pattern defining an amount of corneal tissue to be ablated, wherein the ablation pattern is obtained on the basis of a predetermined amount of at least one of sphere, spherical aberration, coma, and astigmatism that the eye shall have after the eye has been ablatingly treated in accordance with the ablation pattern.
Induction of a certain amount of sphere, spherical aberration and/or coma and/or astigmatism means that, on purpose, the eye exhibits a longitudinal distribution of focal positions after the ablation process. Rather than defining an ablation pattern that results in a single focal point at or near the retina of the eye, embodiments of the invention achieves a focal range that is elongated in the direction of the optical axis of the eye, wherein in this range the focal dimensions are smaller than a pre-defined maximum value. This focal range may be referred to as a "focal tube".

According to embodiments of the present disclosure, an ablation pattern is determined for an individual patient's eye and ablation is then performed in according with the determined ablation pattern using a conventional LASIK technique. In this way, an optimum compromise can be achieved between visual acuity and depth of focus. This is achieved by intentionally inducing, through refractive laser surgery, specific amounts of aberration to the eye, wherein the aberration include at least sphere, spherical aberration, coma, and/or astigmatism. According to embodiments of the present invention, higher-order aberrations (HOA) may be induced in addition to the afore-mentioned types of lower-order aberration.
For example, the total visual comfort of the patient can be improved by accepting a loss in visual acuity of about 0,8 (visus) with regard to one eye and, at the same time, improve the depth of field by about 1-3 diopters. This can result in a total improvement of visual acuity (for both eyes) of about 40%.
According to embodiments of the invention, the dependency between visual acuity and depth of focus is utilized such that a well-defined certain amount of sphere, spherical aberration and/or coma (optionally also using HOA) is maintained in order to treat presbyopia, i.e. to obtain an increased depth of focus and, in particular, improved vision in the near range. The calculated ablation pattern may account for potential future changes of the amount of presbyopia of the pertinent eye.
Also, the mutual correlation between coma and astigmatism and/or sphere and spherical aberration can be used because they often compensate each other.

The determination of the ablation pattern and, thereby, generation of the focus tube is performed as follows. Based on parameters of the individual eye of the patient, as outlined above, an individual model of the patient's eye is generated. This procedure is known in the art. A focus tube of selected diameter and length is introduced into the individual eye model. The ray tracing technique is used to define the focus tube on the basis of the selection of a sphere, spherical aberration, coma, and/or astigmatism. The parameters are, by calculation, changed until a suitable focus tube length and focus tube diameter is obtained which results in a wanted depth of field.

In a normal eye, ideally, the light is bundled such that a sharp image is generated in the layer of photo-receptors of the retina. Geometrically, therefore, the focus must be located directly in front of that layer and, during accommodation, the refractive power is moved back and forth to bring objects in various distances in focus.

The human eye usually applies two different mechanisms for accommodation in the near field, namely the accommodation and the pseudo accommodation. Accommodation is an active change of the form of the lens. The ability of the lens to deform decreases during lifetime to typically 0 diopters at an age of about 50 years. The pseudo accommodation results from pin hole effects (depth of field) but is also caused by High Order Aberrations (HOA) in the cornea and in the lens. By fixing an object in the near field the pupil becomes smaller and in connection with spherical aberrations (prolate cornea, old lens) the eye becomes somewhat near-sighted and thus the patient achieves improved visual acuity in the near field. The effect obtained by embodiments of the present invention can be understood in the sense of pseudo accommodation. The improved accommodation is in the range of about 1 to 3 diopters. Based on pre-measured optical aberrations, when indicated including HOA, of an eye of a patient purposely an ablation pattern is calculated by ray tracing and mixing certain amounts of sphere, spherical aberrations, astigmatism, and coma such that a compromise can be achieved that is agreed upon by the patient. That compromise is achieved by improved pseudo accommodation by the expense of a somewhat reduced visual acuity (visus). The optimum compromise can be achieved, in special cases, by using adaptive optics in order to test the individual patient's perception.

Once the ablation pattern is calculated as described above, the patient's eye can be treated according to well-known LASIK-technologies or PRK.

According to an embodiment of the invention, the ablation pattern is determined also on the basis of the following measured parameters, namely the pre-operative refraction (sphere and cylinder), the corneal tomography (using, e.g., a WaveLight Oculyzer), the pre-operative aberration (using e.g., a WaveLight Analyzer), geometrical data of the eye (length of bulbous, thickness of the lens), data of the pupil (mesopic and photopic pupil, near reflex).

By using the afore-described technology, a platform is provided that can be used by the ophthalmologist and the patient and by using pre-operatively adaptive optics, an optimum compromise between depth of focus (in diopters) and a certain loss of visual acuity or distance. To achieve about two diopters of pseudo accommodation the "length" of the focus tube (Rayleigh length) is about 1.2 mm.

Adaptive optics for studying visual functions are described in a paper of Austin Roorda, Journal of Vision (2011) 11(5): 6, 1-21.

Visual acuity under combined astigmatism and coma are studied by de Gracia, P., Dorronsoro, C., Marin, G., Hernández, M., & Marcos, S. (2011). Visual acuity under combined astigmatism and coma: Optical and neural adaptation effects. Journal of Vision, 11(2): 5, 1-11.

Rocha, Vabre, Chateau, and Krueger describe expanding depth of focus by modifying higher-order aberrations induced by an adaptive optics visual simulator in J Cataract Refract Surg 2009; 35: 1885-1892.

In the following a system and method of the invention are described with regard to embodiments shown in the figures.
- Figur 1: shows, schematically, a system for reshaping a cornea;
- Figur 2A: shows, in expanded presentation, the focussing of light within the human eye near the retina; and
- Figur 2B: shows the focus tube achieved in accordance with embodiments of the present invention near the retina.

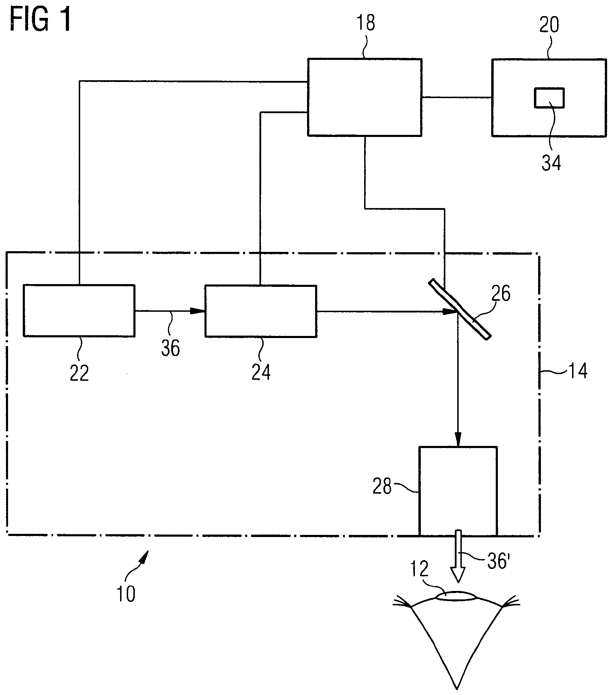

According to figure 1, a system 10 for reshaping a cornea 12 of a patient's eye to treat presbyopia comprises a laser device 14, a control computer 18, and a memory 20 coupled as exemplary shown. The laser device 14 includes a laser source 22, a scanner 24, one or more optical mirrors 26, and a focussing objective 28 coupled as exemplary shown. The memory 20 stores a computer program for controlling optical means, in particular the laser source 22, the scanner 24, one or more optical mirrors 26, and the focusing objective 28.
The laser source 22 generates laser radiation 36 which, under control by computer 18, is guided and focussed onto the cornea 12 of the eye. The scanning is performed in accordance e.g., with LASIK technology in order to ablate corneal tissue to reshape the cornea. An ablation pattern is stored in program 34 and the computer 18 controls the focused laser radiation 36' onto the eye in accordance with the ablation pattern to treat, in the present case, presbyopia. The laser source is, typically, an excimer laser.
The computer program 34 stored in memory 20 comprises, inter alia, an ablation pattern defining the corneal tissue to be ablated (removed) from the cornea 12.
The ablation pattern is generated by first measuring at least the following parameters of the eye to be treated: pre-operative refraction (sphere, cylinder), corneal tomography, using e.g., an WaveLight Oculyzer, pre-operative aberrations, using e.g., an WaveLight Analyser, geometrical data of the eye, in particular the length of the bulbous, the thickness of the lens, and data regarding the pupil, in particular data regarding the mesopical and the photopical pupil, and regarding the reflex in the near field.
Based on such measured data, as is principally known in the art, by ray tracing technique, an ablation pattern is calculated for the individual eye of the patient. According to the disclosure, only one eye of the patient is treated as described here, whereas the other eye may be treated in a different manner.

The ablation pattern, in accordance with this example is not calculated such that the final properties of the eye achieved after the refractive surgery are as close as possible to an ideal eye. Rather, purposely, the ablation pattern is calculated such that certain aberrations remain in the cornea as outlined above. The ablation pattern is such that, after ablation, the optical properties of the eye comprise a predetermined amount of sphere and/or spherical aberration and/or coma, and/or astigmatism. Depending on the measured data of the eye, the ablation pattern can be calculated such that higher-order aberrations (HOA) also remain after surgery. The so-calculated ablation pattern is used by the system of figure 1 to effect ablation of the cornea 12.

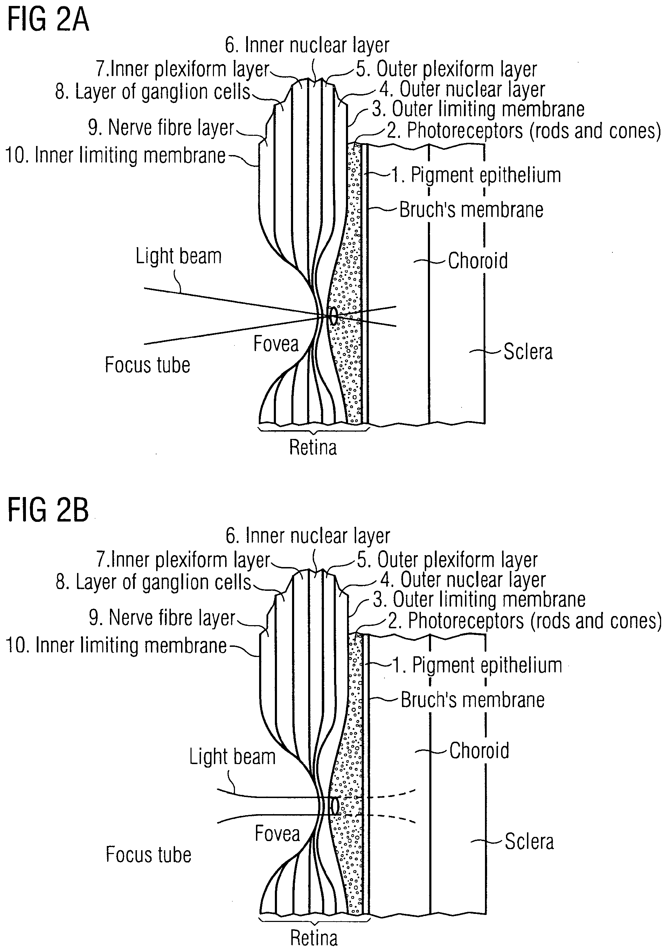

Figures 2A and 2B illustrate the effect achieved when applying the afore-described methods of inducing a predetermined amount of aberration into a presbyopic human eye. Figure 2A shows the focussing of light within the eye near the retina without the induced "focal tube" as described above. Figure 2B shows the induced "focal tube" in accordance with the above-described embodiments of the present invention. The anatomical details are indicated in the figures.

## Claims

1. A method of determining a corneal ablation pattern for treating presbyopia of a human eye, comprising:
- defining a non-zero amount of induced aberration for a presbyopic human eye; and
- applying a ray tracing process based on an eye model in conjunction with measured data of the eye to be treated to determine a corneal ablation pattern defining an amount of corneal tissue that, when removed from the eye, leaves the eye with the defined amount of induced aberration,
**characterized in that**:
defining the non-zero amount of induced aberration includes introducing into the eye model a focus tube of selected diameter and length;
the induced aberration is effective to increase a depth of focus of the eye by about 1 to 3 diopters at least essentially by way of pseudo accommodation, and
the ray tracing process is performed using measurement data for pre-operative refraction of the eye, pre-operative aberrations of the eye, corneal topography of the eye, one or more opto-geometrical parameters of the eye, and one or more pupil-related parameters, and the ray tracing process is used to determine the corneal ablation pattern for obtaining the focus tube.

2. The method of claim 1, wherein the induced aberration includes at least one of sphere, spherical aberration, coma, astigmatism and/or a combination of these aberrations.

3. The method of claim 2, wherein the induced aberration further includes higher-order aberrations.

4. A method of generating a control program for an ophthalmic laser apparatus, the control program containing instructions that, when executed by a control computer of the apparatus, control an optical system of the apparatus to move focused laser radiation over a target, the method comprising steps of:
- defining a non-zero amount of induced aberration for a presbyopic human eye;
- applying a ray tracing process based on an eye model in conjunction with measured data of the eye to be treated to determine a corneal ablation pattern defining an amount of corneal tissue that, when removed from the eye, leaves the eye with the defined amount of induced aberration; and
- generating instructions for the computer program in accordance with the determined ablation pattern,
**characterized in that**:
defining the non-zero amount of induced aberration includes introducing into the eye model a focus tube of selected diameter and length;
the induced aberration is effective to increase a depth of focus of the eye by about 1 to 3 diopters at least essentially by way of pseudo accommodation, and the ray tracing process is performed using measurement data for pre-operative refraction of the eye, pre-operative aberrations of the eye, corneal topography of the eye, one or more opto-geometrical parameters of the eye, and one or more pupil-related parameters, and the ray tracing process is used to determine the corneal ablation pattern for obtaining the focus tube.

5. A computer program product containing instructions that, when executed by a computer,
- perform a ray tracing process based on an eye model in conjunction with measured data of the eye to be treated to determine a corneal ablation pattern defining an amount of corneal tissue that, when removed from a presbyopic human eye, leaves the eye with a pre-defined non-zero amount of induced aberration; and
- control a laser device to perform ablation on the eye in accordance with the determined ablation pattern,
**characterized in that**:
the induced aberration corresponds to a focus tube of selected diameter and length which has been introduced into the eye model, and the induced aberration is effective to increase a depth of focus of the eye by about 1 to 3 diopters at least essentially by way of pseudo accommodation, and
the ray tracing process is performed using measurement data for pre-operative refraction of the eye, pre-operative aberrations of the eye, corneal topography of the eye, one or more opto-geometrical parameters of the eye, and one or more pupil-related parameters, and the ray tracing process is used to determine the corneal ablation pattern for obtaining the focus tube.

## Patentansprüche

1. Verfahren zum Bestimmen eines Hornhautablationsmusters zur Behandlung von Presbyopie eines menschlichen Auges, umfassend:
- Definieren eines Nicht-Null-Betrags einer induzierten Aberration für ein presbyopes menschliches Auge und
- Anwenden eines Strahlverfolgungsprozesses auf der Grundlage eines Augenmodells in Zusammenhang mit Messdaten des zu behandelnden Auges, um ein Hornhautablationsmuster zu bestimmen, das einen Betrag an Hornhautgewebe definiert, dessen Entfernung aus dem Auge dazu führt, dass das Auge den definierten Betrag der induzierten Aberration aufweist,
**dadurch gekennzeichnet, dass**:
Definieren des Nicht-Null-Betrags von induzierter Aberration Einführen einer Fokusröhre mit ausgewähltem Durchmesser und ausgewählter Länge in das Augenmodell beinhaltet;
die induzierte Aberration wirksam ist, eine Schärfentiefe des Auges um ungefähr 1 bis 3 Dioptrien mindestens im Wesentlichen mittels Pseudoakkomodation zu erhöhen, und
der Strahlverfolgungsprozess mittels Messdaten für präoperative Brechung des Auges, präoperative Aberrationen des Auges, Hornhauttopographie des Auges, ein oder mehr optogeometrische Parameter des Auges und ein oder mehr pupillenbezogene Parameter durchgeführt wird, und der Strahlverfolgungsprozess zur Bestimmung des Hornhautablationsmusters zum Erhalten der Fokusröhre verwendet wird.

2. Verfahren nach Anspruch 1, wobei die induzierte Aberration mindestens eine von Sphäre, sphärische Aberration, Koma, Astigmatismus und/oder eine Kombination von diesen Aberrationen beinhaltet.

3. Verfahren nach Anspruch 2, wobei die induzierte Aberration weiterhin Aberrationen höherer Ordnung beinhaltet.

4. Verfahren zum Erzeugen eines Steuerprogramms für eine ophthalmische Laservorrichtung, wobei das Steuerprogramm Anweisungen enthält, die bei Ausführung durch einen Steuercomputer der Vorrichtung ein optisches System der Vorrichtung dazu ansteuern, fokussierte Laserstrahlung über ein Ziel zu bewegen, wobei das Verfahren die folgenden Schritte umfasst:
- Definieren eines Nicht-Null-Betrags einer induzierten Aberration für ein presbyopes menschliches Auge;
- Anwenden eines Strahlverfolgungsprozesses auf der Grundlage eines Augenmodells in Zusammenhang mit Messdaten des zu behandelnden Auges, um ein Hornhautablationsmuster zu bestimmen, das einen Betrag an Hornhautgewebe definiert, dessen Entfernung aus dem Auge dazu führt, dass das Auge den definierten Betrag der induzierten Aberration aufweist; und
- Erzeugen von Anweisungen für das Computerprogramm gemäß dem bestimmten Ablationsmuster,
**dadurch gekennzeichnet, dass**:
Definieren des Nicht-Null-Betrags von induzierter Aberration Einführen einer Fokusröhre mit ausgewähltem Durchmesser und ausgewählter Länge in das Augenmodell beinhaltet;
die induzierte Aberration wirksam ist, eine Schärfentiefe des Auges um ungefähr 1 bis 3 Dioptrie mindestens im Wesentlichen mittels Pseudoakkomodation zu erhöhen, und
der Strahlverfolgungsprozess mittels Messungsdaten für präoperative Brechung des Auges, präoperative Aberrationen des Auges, Hornhauttopographie des Auges, ein oder mehr optogeometrische Parameter des Auges und ein oder mehr pupillenbezogene Parameter durchgeführt wird, und der Strahlverfolgungsprozess zur Bestimmung des Hornhautablationsmusters zum Erhalten der Fokusröhre verwendet wird.

5. Computerprogrammprodukt, das Anweisungen enthält, die bei Ausführung durch einen Computer
- einen Strahlverfolgungsprozess auf der Grundlage eines Augenmodells in Zusammenhang mit Messdaten des zu behandelnden Auges durchführen, um ein Hornhautablationsmuster zu bestimmen, das einen Betrag an Hornhautgewebe definiert, dessen Entfernung aus einem presbyopen menschlichen Auge dazu führt, dass das Auge einen vordefinierten Nicht-Null-Betrag an induzierter Aberration aufweist; und
- eine Lasereinrichtung dazu ansteuern, Ablation am Auge gemäß dem vorbestimmten Ablationsmuster durchzuführen,
**dadurch gekennzeichnet, dass**:
die induzierte Aberration einer Fokusröhre mit ausgewähltem Durchmesser und ausgewählter Länge, die in das Augenmodell eingeführt wurde, entspricht, und die induzierte Aberration wirksam ist, eine Schärfentiefe des Auges um ungefähr 1 bis 3 Dioptrie mindestens im Wesentlichen mittels Pseudoakkomodation zu erhöhen, und
der Strahlverfolgungsprozess mittels Messungsdaten für präoperative Brechung des Auges, präoperative Aberrationen des Auges, Hornhauttopographie des Auges, ein oder mehr optogeometrische Parameter des Auges und ein oder mehr pupillenbezogene Parameter durchgeführt wird, und der Strahlverfolgungsprozess zur Bestimmung des Hornhautablationsmusters zum Erhalten der Fokusröhre verwendet wird.

## Revendications

1. Procédé de détermination d'un motif d'ablation cornéenne destiné à traiter une presbytie d'un oeil humain, comprenant :
- la définition d'une quantité non nulle d'aberrations induites pour un oeil humain presbyte ; et
- l'application d'un processus de lancer de rayon basé sur un modèle d'oeil conjointement avec des données mesurées de l'oeil à traiter pour déterminer un motif d'ablation cornéenne définissant une quantité de tissu cornéen qui, une fois enlevé de l'oeil, laisse l'oeil avec la quantité définie d'aberrations induites,
**caractérisé en ce que** :
la définition de la quantité non nulle d'aberrations induites inclut l'introduction dans le modèle d'oeil d'un tube de foyer de diamètre et de longueur sélectionnés ;
l'aberration induite est efficace pour augmenter une profondeur de foyer de l'oeil d'environ 1 à 3 dioptres au moins essentiellement au moyen d'une pseudo-accommodation, et
le processus de lancer de rayon est réalisé en utilisant des données de mesure pour une réfraction préopératoire de l'oeil, des aberrations préopératoires de l'oeil, une topographie cornéenne de l'oeil, un ou plusieurs paramètres optogéométriques de l'oeil, et un ou plusieurs paramètres relatifs à la pupille, et le processus de lancer de rayon est utilisé pour déterminer le motif d'ablation cornéenne pour obtenir le tube de foyer.

2. Procédé selon la revendication 1, dans lequel l'aberration induite inclut au moins l'une d'une sphère, d'une aberration sphérique, d'une coma, d'un astigmatisme et/ou d'une combinaison de ces aberrations.

3. Procédé selon la revendication 2, dans lequel l'aberration induite inclut en outre des aberrations d'ordre supérieur.

4. Procédé de génération d'un programme de commande pour un appareil laser ophtalmique, le programme de commande contenant des instructions qui, lorsqu'elles sont exécutées par un ordinateur de commande de l'appareil, commande un système optique de l'appareil pour déplacer un rayonnement laser focalisé sur une cible, le procédé comprenant les étapes :
- de définition d'une quantité non nulle d'aberrations induites pour un oeil humain presbyte ; et
- d'application d'un processus de lancer de rayon basé sur un modèle d'oeil conjointement avec des données mesurées de l'oeil à traiter pour déterminer un motif d'ablation cornéenne définissant une quantité de tissu cornéen qui, une fois enlevé de l'oeil, laisse l'oeil avec la quantité définie d'aberrations induites ; et
- de génération d'instructions pour le programme d'ordinateur en conformité avec le motif d'ablation déterminé,
**caractérisé en ce que** :
la définition de la quantité non nulle d'aberrations induites inclut l'introduction dans le modèle d'oeil d'un tube de foyer de diamètre et de longueur sélectionnés ;
l'aberration induite est efficace pour augmenter une profondeur de foyer de l'oeil d'environ 1 à 3 dioptres au moins essentiellement au moyen d'une pseudo-accommodation, et
le processus de lancer de rayon est réalisé en utilisant des données de mesure pour une réfraction préopératoire de l'oeil, des aberrations préopératoires de l'oeil, une topographie cornéenne de l'oeil, un ou plusieurs paramètres optogéométriques de l'oeil, et un ou plusieurs paramètres relatifs à la pupille, et le processus de lancer de rayon est utilisé pour déterminer le motif d'ablation cornéenne pour obtenir le tube de foyer.

5. Produit programme d'ordinateur contenant des instructions qui, lorsqu'elles sont exécutées par un ordinateur,
- réalisent un processus de lancer de rayon basé sur un modèle d'oeil conjointement avec des données mesurées de l'oeil à traiter pour déterminer un motif d'ablation cornéenne définissant une quantité de tissu cornéen qui, une fois enlevé d'un oeil humain presbyte, laisse l'oeil avec une quantité non nulle prédéfinie d'aberrations induites ; et
- commandent un dispositif laser pour réaliser une ablation sur l'oeil en conformité avec le motif d'ablation déterminé,
**caractérisé en ce que** :
l'aberration induite correspond à un tube de foyer de diamètre et de longueur sélectionnés qui a été introduit dans le modèle d'oeil, et l'aberration induite est efficace pour augmenter une profondeur de foyer de l'oeil d'environ 1 à 3 dioptres au moins essentiellement au moyen d'une pseudo-accommodation, et
le processus de lancer de rayon est réalisé en utilisant des données de mesure pour une réfraction préopératoire de l'oeil, des aberrations préopératoires de l'oeil, une topographie cornéenne de l'oeil, un ou plusieurs paramètres optogéométriques de l'oeil, et un ou plusieurs paramètres relatifs à la pupille, et le processus de lancer de rayon est utilisé pour déterminer le motif d'ablation cornéenne pour obtenir le tube de foyer.
